# EUROPEAN PATENT APPLICATION

(11) **EP 4 527 916 A1**
(43) Date of publication of application: **26.03.2025**
(21) Application number: 23807281.3
(22) Date of filing: 24.03.2023
(51) Int. Cl.: C12M 3/00, C12N 5/071, C12N 5/077, C12N 5/0793

(54) **CELL CULTURE DEVICE AND CELL CULTURE METHOD**

(30) Priority: 19.05.2022 JP 2022082309
(71) Applicant: Kyushu Institute of Technology, Kitakyushu-shi Fukuoka 804-8550 (JP)
(72) Inventor: YASUDA Takashi, Kitakyushu-shi, Fukuoka 808-0196 (JP); NAKAMA Ayaka, Kitakyushu-shi, Fukuoka 808-0196 (JP)
(74) Representative: TBK
(86) International application number: PCT/JP2023/011681
(87) International publication number: WO 2023/223666

(57) **Abstract**

A cell culture device 1 comprising a lower basal plate 10 comprising a cell culture part 14 having a bottom membrane, an upper basal plate 12 arranged on an upper side of the lower basal plate 10, and comprising a cell housing part 24 having a bottom membrane in which a micropore is formed, wherein when arranging the upper basal plate 12 on the lower basal plate 10, the bottom membrane of the lower basal plate 10 and the bottom membrane of the upper basal plate 12 are arranged spaced apart up and down by a predetermined distance; and a cell culture method using the cell culture device 1.

## Description

### Technical Field

The present invention relates to a cell culture device and a cell culture method using the cell culture device.

### Background Art

Conventionally, in the field of medicine or drug development, cells are cultured and analyzed in cell culture devices to elucidate structure and function of various types of cells, tissues, organs in vivo. As analysis methods using such cell culture devices, for example, a method for high-throughput analysis of cellular functions at the single-cell level by forming an array of many minute cell culture sections using microfabrication technique based on semiconductor processing technique and culturing a single cell in each cell culture section, a method of analyzing cell-to-cell communication by co-culturing various types of cells in minute cell culture sections formed by microfabrication technique, and further a method of analyzing pharmacokinetics, etc. by using tissues or organs reconstructed in minute cell culture sections are known.

As cell culture device used in such analysis method, for example, a cell culture device wherein each well bottom surface of microwell array is constituted of self-supported membrane made by SiN by semiconductor processing technique, and multiple micro through-holes having a diameter of about 3 µm are formed on the SiN membrane is proposed (see non Patent Literature 1). By culturing multiple astrocytes on the back surface of the SiN membrane, and a single neuron on the surface of SiN membrane in each well, cell-to-cell communication through micro-through holes became possible, and it has been succeeded to maintain for a long period the physiological activity of a single neuron.

However, when seeding directly cells to microwell array using micropipette, the probability of introducing one cell into one well was low, and it was very difficult. **In** case of making the size of the well as small as about the cell size, since physically two or more cells can hardly be introduced in one well, and the success rate would be relatively high. However, in case of culturing adherent cells that require a large culture surface area such as neuron, the size of the well being necessary large, and it was difficult to introduce one cell in one well, and the success was about a few %.

Further, a double layer micro-flow channel device separated by porous membrane made by semiconductor processing technique is proposed (see Patent Literature 1). In the first micro-flow channel, a retinal layer is formed by culturing retinal pigment epithelium cells, and in the second micro-flow channel, a blood vessel layer is formed by culturing vascular endothelial cells and fibroblast, and thereby a model of blood-retinal barrier consisting of two layers is formed.

However, by applying such double layer type micro flow channel device separated by a porous membrane, to form tissues having three or more layers, it was necessary to provide two or more porous membranes in the micro flow channel. Further, such device is made by sticking the upper and lower micro flow channels produced by semiconductor processing technique and the porous membrane consisting of plastic material such as polyethylene terephthalate (PET), and when the number of layer increases, the positioning accuracy when sticking worsens. Further, the distance between layers was hard to control, and for example, even if the cells of the first layer and the second layer could be arranged in the near distance separated by the porous membrane, it was difficult to get closer the cells of the second layer and the third layer to an appropriate distance.

### Citation List

### Patent Literature

[Patent Literature 1] Japanese Patent Laid-Open Publication No. 2020-188723

### Non-Patent Literature

[Non-Patent Literature 1]
Yusuke Zenmyo, Isamu Morisako, Takashi Yasuda; Long-term Culture of a Single Neuron using a Microwell with a Porous SiN Membrane, Article of The Institute of Electrical Engineers of Japan E, Vol. 138, No.7, pp. 327-328 (2018)

### Summary of Invention

### Technical Problem

The object of the present invention is to provide a new cell culture device, and a culture method using such cell culture device.

### Solution to Problem

The present inventors found a new cell culture device useful for analysis of function at a single cell level, or analysis of cell-to-cell communication, etc. and a culture method using the cell culture device. The present invention has been thus completed.

Specifically, the present invention relates to the following.
[1] A cell culture device comprising:
   a lower basal plate comprising a cell culture part having a bottom membrane, and
   an upper basal plate arranged on an upper side of the lower basal plate, and comprising a cell housing part having a bottom membrane in which a micropore is formed,
   wherein when arranging the upper basal plate on the lower basal plate, the bottom membrane of the lower basal plate and the bottom membrane of the upper basal plate are arranged spaced apart up and down by a predetermined distance.
[2] The cell culture device according to [1], wherein one micropore is formed in the bottom membrane of the upper basal plate.
[3] The cell culture device according to [2], wherein a plurality of micropores are formed in the bottom membrane of the lower basal plate.
[4] The cell culture device according to [2] or [3], wherein the bottom membrane of the upper basal plate and/or the lower basal plate is constituted of a transparent material.
[5] The cell culture device according to [4], wherein the bottom membrane of the upper basal plate and/or the lower basal plate comprises at least one inorganic material selected from silicon nitride (SiN), silicon oxide (SiO₂), and silicon oxynitride (SiON).
[6] The cell culture device according to any one of [2] to [5], wherein the lower basal plate comprises a convex part or a concave part, and the upper basal plate comprises a concave part or a convex part, wherein the convex part or the concave part of the lower basal plate and the concave part or the convex part of the upper basal plate are mated to perform position adjustment of the bottom membrane of the lower basal plate and the bottom membrane of the upper basal plate.
[7] The cell culture device according to any one of [2] to [6], wherein the lower basal plate comprises a plurality of cell culture parts arranged in a grid pattern, and the upper basal plate comprises a plurality of cell housing parts arranged in a grid pattern.
[8] The cell culture device according to [7], wherein the upper part of a lower partitioning member that divides the plurality of grid-patterned cell culture parts of the lower basal plate constitutes a convex part, and a concave part corresponding to the upper convex part of the lower partitioning member is formed on the lower part of an upper partitioning member, corresponding to the lower partitioning member, that divides the plurality of cell housing parts of the upper basal plate.
[9] The cell culture device according to [1], wherein a plurality of micropores are formed in the bottom membrane of the cell culture part of the lower basal plate, and a plurality of micropores are formed in the bottom membrane of the cell housing part of the upper basal plate.
[10] The cell culture device according to [9], wherein the bottom membrane of the upper basal plate and/or the lower basal plate is constituted of a transparent material.
[11] The cell culture device according to [10], wherein the bottom membrane of the upper basal plate and/or the lower basal plate comprises at least one inorganic material selected from silicon nitride (SiN), silicon oxide (SiO₂), and silicon oxynitride (SiON).
[12] The cell culture device according to any one of [9] to [11], wherein the bottom membrane of the cell culture part of the lower basal plate and the bottom membrane of the cell housing part of the upper basal plate are arranged spaced apart up and down by a distance of 5 to 500 µm.
[13] The cell culture device according to any one of [9] to [12], wherein the lower basal plate comprises a convex part or a concave part, and the upper basal plate comprises a concave part or a convex part, wherein the convex part or the concave part of the lower basal plate and the concave part or the convex part of the upper basal plate are mated to perform position adjustment of the bottom membrane of the lower basal plate and the bottom membrane of the upper basal plate.
[14] The cell culture device according to any one of [9] to [13], wherein the lower basal plate comprises a plurality of cell culture parts arranged in a grid pattern, and the upper basal plate comprises a plurality of cell housing parts arranged in a grid pattern.
[15] The cell culture device according to [14], wherein the upper part of a lower partitioning member that divides the plurality of the grid-patterned cell culture parts of the lower basal plate constitutes a convex part, and a concave part corresponding to the upper convex part of the lower partitioning member is formed on the lower part of an upper partitioning member, corresponding to the lower partitioning member, that divides the plurality of cell housing parts of the upper basal plate.
[16] The cell culture device according to any one of [1] to [15], wherein the lower basal plate and the upper basal plate are fabricated by using photolithography which is a semiconductor processing technique.
[17] A cell culture method comprising culturing cells by using the cell culture device according to any one of [2] to [8], and [16], comprising seeding cells on the bottom membrane of the cell housing part of the upper basal plate, allowing a cell to drop on the bottom membrane of the cell culture part of the lower basal plate, through one micropore formed in the bottom membrane of the cell housing part of the upper basal plate, to culture the cell at the cell culture part.
[18] A cell culture method comprising culturing cells by using the cell culture device according to any one of [9] to [16], comprising culturing cells on one surface or both surfaces of the cell culture membrane of the lower basal plate, and culturing cells on one surface or both surfaces of the cell culture membrane of the upper basal plate.

### Advantageous Effects of Invention

According to the present invention, a new cell culture device useful for analysis of function at a single cell level, or analysis of cell-to-cell communication, and a cell culture method can be provided.

### Brief Description of Drawing

[Figure 1] It is a brief explanation drawing of a cell culture device of one embodiment of the first invention.
[Figure 2] It is a brief cross-section view of the lower basal plate and the upper basal plate of the cell culture device of Fig. 1.
[Figure 3] It is an explanation drawing of the cell culture method using the cell culture device of Fig. 1.
[Figure 4] It is a brief explanation drawing of a cell culture device of one embodiment of the second invention.
[Figure 5] It is a brief cross-section view of the lower basal plate and the upper basal plate of the cell culture device of Fig. 4
[Figure 6] It is an explanation drawing of the cell culture method using the cell culture device of Fig. 4.
[Figure 7] It is a photograph of a cell culture device produced in the Example, and (a) shows the upper basal plate, and (b) shows the lower basal plate.
[Figure 8] It is a result of cell seeding test using the cell culture device produced in the Example, and it is a figure showing "the number of wells" based on "the number of cells per well" (for example, the number of wells in which one cell was introduced).

### Description of Embodiments

The cell culture device of the present invention is characterized by comprising a lower basal plate comprising a cell culture part having a bottom membrane, and an upper basal plate arranged on the upper side of the lower basal plate, and comprising a cell housing part having a bottom membrane in which a micropore are formed. When arranging the upper basal plate on the lower basal plate, the bottom membrane of the lower basal plate and the bottom membrane of the upper basal plate are arranged spaced apart up and down by a predetermined distance. The cell culture device of the present invention is not limited to those wherein the basal plates are arranged in two layers one above the other, and can be one wherein three or more layers are stacked.

The cell culture device of the present invention is useful for analysis of function at a single cell level, or analysis of cell-to-cell communication, etc.

Cells being the subject of culture of the cell culture device of the present invention are not particularly limited, and can be appropriately selected according to the purpose. For example, cells forming tissues such as skin, retina, cardiac muscle, blood vessel, nerve, organs, etc. collected from human body or animals; cell lines established from them; stem cells such as, mesenchymal stem cells (MSC), induced pluripotent stem cells (iPS cells), embryo stem cells (ES cells); cells forming tissues such as nerve, skin, cardiac muscle, liver, etc., induced to differentiate from stem cells can be exemplified.

### <Cell culture device of the first invention>

In the following, the cell culture device of the first invention is explained.

The cell culture device of the first invention is characterized by comprising a lower basal plate comprising a cell culture part having a bottom membrane, and an upper basal plate arranged on the upper side of the lower basal plate, and comprising a cell housing part having a bottom membrane in which one micropore is formed, and when arranging the upper basal plate on the lower basal plate, the bottom membrane of the lower basal plate and the bottom membrane of the upper basal plate are arranged spaced apart up and down by a predetermined distance.

The cell culture device of the first invention can seed a single cell into the cell culture part of the lower basal plate at a high probability by allowing a cell to drop through one micropore formed in the bottom membrane of the cell housing part of the upper basal plate. Specifically, as compared to when conventionally seeding directly cells using a micropipette, it is possible to introduce only one cell into one cell culture part (well) at a high probability. Further, even when the surface area of the cell culture part is large, it is possible to introduce one cell at a high probability, and it can be suitably used for culture of adherent cells that require a large culture surface area, such as neurons.

In the following, each member of the cell culture device of the first invention is explained.

### [Lower basal plate]

The lower basal plate is a plate comprising the cell culture part, and as material, for example, silicon wafer, glass wafer, and ceramic wafer such as quartz wafer can be exemplified. The thickness is preferably 200 µm or more, more preferably 200 to 500 µm, and further preferably 250 to 350 µm.

### (Cell culture part)

The cell culture part is a portion where the cells are seeded and cultured, and for example, it is a well (depressed area) formed in the lower basal plate. The shape of the inner space of the cell culture part is not particularly limited, and for example, shapes such as cylindrical shape, rectangular column shape, inverted truncated cone shape, inverted truncated pyramid shape, etc. can be exemplified. Inverted truncated pyramid shape is preferable, and inverted truncated quadrangular shape (inverted pyramid shape) is particularly preferable. One or more cell culture parts can be provided, and generally, they have a number corresponding to the number of cell housing parts of the upper basal plate, described in the following, and they are arranged in a position corresponding to the cell housing parts of the upper basal plate. The cell culture parts are preferably arranged in a large number in a grid pattern (array pattern).

In case the cell culture parts are arranged in a grid pattern, the cell culture parts are separated and comparted by a grid-patterned partitioning member (lower partitioning member) that constitutes the side walls (peripheral walls) of the cell culture parts. It is preferable that the concave portion formed on the lower part of the upper partitioning member of the cell housing parts of the upper basal plate described in the following is mated to all or a part of the upper part of the lower partitioning member, to perform position adjustment of the cell culture parts of the lower basal plate and the cell housing parts of the upper basal plate.

The size (diameter (diagonal line in case of polygon)) of the bottom membrane (cell culture membrane) of the cell culture part is preferably 100 to 1000 µm, more preferably 150 to 800 µm, and further preferably 200 to 600 µm.

Micropores may not be formed in the cell culture membrane, while it is preferable that a plurality of micropores are formed. Thereby, for example, in case cells are loaded on one of the surfaces of the cell culture membrane, it is possible to supply nutrient of the medium through micropores from the other surface. Further, in case cells are loaded on both surfaces of the cell culture membrane, it is possible to smoothly perform cell-to-cell communication via micropores.

The shape of micropores can be appropriately set according to types of cells or purpose of the culture, and for example, shapes such as circular shape, polygonal shape can be exemplified. The diameter of the micropores (diagonal line in case of polygon) is preferably a size that cells cannot pass through, preferably 20 µm or less, more preferably 0.1 to 10 µm, further preferably 0.5 to 5 µm, and particularly preferably 1 to 4 µm. The area ratio of micropores on the bottom membrane of the cell culture part depends on the diameter of the micropores, but is preferably 10 to 70%, more preferably 10 to 50 %, and further preferably 10 to 20%. If it is within this range, supply of nutrient through micropores, or cell-to-cell communication can be smoothly performed, and in case it is a transparent material, high transparency can be maintained.

The membrane thickness of the cell culture membrane is preferably, for example 0.1 to 5 µm, more preferably 0.5 to 1.5 µm. If it is within this range, the mechanical strength as cell scaffold can be maintained, and in case it is a transparent material, high transparency can be maintained.

The material of the cell culture membrane is not particularly limited as long as it can be a cell scaffold, while from the viewpoint that cell observation is facile, it is preferable to be composed of transparent material. As transparent material, it can be an organic material, while inorganic material is preferable. Inorganic materials include, for example, those that are transparent, among nitride, oxide, oxynitride, etc. of silicon, titanium, zinc, tin, and aluminum. Specifically, silicon nitride (SiN), silicon oxide (SiO₂), titanium oxide (TiO₂), zinc oxide (ZnO), tin oxide (SnO₂), silicon oxynitride (SiON), titanium oxynitride (TiON), indium oxide (In₂O₃), indium tin oxide (ITO), etc. can be exemplified. Silicon nitride, silicon oxide, silicon oxynitride are preferable, and silicon nitride or silicon oxide is particularly preferable. These transparent inorganic materials can be used in combination of two or more. Further, a laminated membrane consisting of two or more types can be formed. For example, it can be an inorganic material with a transparent organic material coated on its surface.

Here, in the present specification, "transparent" means that the light transmittance at a wavelength of 500 nm to 600 nm is 70% or more, preferably 80% or more, and more preferably 90% or more.

It is preferable that the surface of the bottom membrane (cell culture membrane) of the cell culture part is modified with functionalized molecules, to promote cell adhesion or cell proliferation. Functionalized molecules are not particularly limited as long as they can be used for cell culture, and they include, for example, extracellular matrix such as collagen, proteoglycan, heparan sulfate proteoglycan, fibronectin, laminin, entactin, elastin, hyaluronic acid, tenascin, etc., cell adhesion peptide such as arginine-glycine-asparagine acid, leucine-asparagine acid-valine, arginine-glutamic acid-asparagine acid-valine, etc., or synthetic molecules such as poly-L-lysine, poly-L-ornithine, etc.

### (Supporting frame part)

It is preferable that the lower basal plate comprises a supporting frame part (holder) supporting the lower basal plate. Thereby, when arranging the upper basal plate on the lower basal plate, it can be arranged with more stability. Further, since it can be used as a grasping part, the operability can be enhanced. As material of the supporting frame part, synthetic resin can be exemplified.

### [Upper basal plate]

The upper basal plate is a plate arranged on the upper side of the above-mentioned lower basal plate, and comprising a cell housing part having a bottom membrane in which one micropore is formed. As material of the basal plate, a similar material as the lower basal plate can be used, and it can be the same material as the lower basal plate, or can be a different material. The thickness of the upper basal plate is preferably 200 µm or more, more preferably 200 to 500 µm, and further preferably 250 to 350 µm.

### (Cell housing part)

The cell housing part is a portion that can house cells, and is for example a well (depressed area) formed in the upper basal plate. The shape of the inner space of the cell housing part is not particularly limited, and for example, shape such as cylindrical shape, rectangular column shape, inverted truncated cone shape, inverted truncated pyramid shape, etc. can be exemplified. Inverted truncated pyramid shape is preferable, and inverted truncated quadrangular shape (inverted pyramid shape) is particularly preferable. One or more cell housing parts can be provided, and generally, they have a number corresponding to the number of cell culture parts of the lower basal plate, described in the above, and they are arranged in a position corresponding to the cell culture parts of the lower basal plate. The cell housing parts are preferably arranged in a large number in a grid pattern (array pattern). In case two or more cell housing parts are provided, each cell housing part has preferably the same constitution, so that cells drop in a uniform manner.

In case the cell housing parts are arranged in a grid pattern, the cell housing parts are separated and comparted by a grid-patterned partitioning member (upper partitioning member) that constitutes the side walls (peripheral walls) of the cell housing parts. As stated above, it is preferable to form a concave part in the lower part of the upper partitioning member, to mate the convex part of the upper part of the lower partitioning member of the cell culture part of the lower basal plate, to perform position adjustment of the two parts. Generally, when arranging the upper basal plate on the lower basal plate, a part of the cell housing part of the upper basal plate is introduced in the cell culture part of the lower basal plate. Therefore, it is preferred that the bottom membrane of the cell housing part of the upper basal plate is smaller than the bottom membrane of the cell culture part of the lower basal plate, for example about 1/3 to 2/3.

In the bottom membrane of the cell housing part, one micropore having a size large enough for a cell to pass through is formed. The one micropore is preferably formed in the center of the bottom membrane of the cell housing part. Thereby, when arranging the upper basal plate on the upper side of the lower basal plate, the micropore is positioned in the center upper side of the bottom membrane of the cell culture part of the lower basal plate, and it is possible to let a cell surely drop into the cell culture part of the lower basal plate. The shape of micropores can be appropriately set according to types of cells, and for example, shapes such as circular shape, polygonal shape can be exemplified. The diameter of the micropores (diagonal line in case of polygon) can be appropriately set according to the types of the cells, and it is preferably larger than a diameter of one cell and smaller than the total of diameters of two cells. For example, it is preferably a diameter of 3 to 50 µm, more preferably 8 to 20 µm. Specifically, for example, in case of neuron, since the diameter is about 8 µm, a micropore having a diameter of 10 to 15 µm is preferable. Thereby, it is possible to seed only one cell into one cell culture part, at high probability.

### (Supporting frame part)

It is preferable that the upper basal plate comprises a supporting frame part (holder) supporting the upper basal plate. Thereby, when arranging the upper basal plate on the lower basal plate, it can be arranged with more stability. Further, since it can be used as a grasping part, the operability can be enhanced. As material of the peripheral wall, synthetic resin can be exemplified.

### [Embodiment of position adjustment of the cell culture part of the lower basal plate and the cell housing part of the upper basal plate]

In the cell culture device of the first invention, when arranging the upper basal plate on the lower basal plate, the bottom membrane of the lower basal plate and the bottom membrane of the upper basal plate are arranged spaced apart up and down by a predetermined distance, and one micropore of the bottom membrane of the upper basal plate is arranged so that it is positioned in the upper side of the bottom membrane of the lower basal plate. At that time, it is preferable that the central parts of the bottom membranes are arranged to coincide with each other. Thereby, it is possible to let cells drop to the bottom membrane of the cell culture part of the lower basal plate reliably and accurately. The distance between the bottom membranes is preferably 5 to 500 µm, more preferably 20 to 250 µm, and further preferably 50 to 200 µm.

The embodiment of position adjustment of the lower basal plate and the upper basal plate is not particularly limited, and its examples include embodiment of mating concave and convex formed on each of the lower basal plate and the upper basal plate; embodiment of adjoining and mating each of the peripheral convex parts having large and small diameters (diagonal line in case of polygon) formed on the outer periphery of each of the lower basal plate and the upper basal plate; embodiment of placing the upper basal plate within the peripheral frame of the lower basal plate; embodiment of aligning the marks added to each of the lower basal plate and the upper basal plate and fixing them with a fixing tool; and embodiment of fixing by inserting the insertion rods to the insertion holes formed in each of the lower basal plate and upper basal plate, etc.

Specifically, as embodiment of mating concave and convex formed on each of the lower basal plate and upper basal plate, for example, its examples include embodiment wherein the lower basal plate comprises a convex part or a concave part, and the upper basal plate comprises a concave part or a convex part, the convex part or the concave part of the lower basal plate and the concave part or the convex part of the upper basal plate are mated to perform position adjustment of the cell culture part of the lower basal plate and the cell housing part of the upper basal plate. More specifically, an embodiment of mating the peripheral concave part or the peripheral convex part surrounding the upper basal plate to the peripheral convex part or the peripheral concave part surrounding the lower basal plate; or an embodiment where the upper part of the lower partitioning member separating a plurality of the grid-patterned cell culture parts of the lower basal plate constitutes the concave part, and a concave part corresponding to the upper convex part of the lower partitioning member is formed on the lower part of the upper partitioning member separating a plurality of cell housing parts of the upper basal plate corresponding to the lower partitioning member, to mate the grid-patterned convex part of the lower basal plate and the grid-patterned concave part of the upper basal plate can be exemplified. One convex part or concave part can be provided on each of the lower basal plate and the upper basal plate, or plural parts can be provided.

As embodiment of adjoining and mating the peripheral convex parts formed on each of the lower basal plate and the upper basal plate, its examples include an embodiment of arranging by adjoining and mating the peripheral convex part surrounding the periphery of the lower basal plate, and the peripheral convex part surrounding the upper basal plate, with a diameter (diagonal line in case of polygon) being one size larger or smaller than the convex part of the lower basal plate.

In case of such embodiment of position adjustment of the lower basal plate and the upper basal plate, the position adjustment of the cell culture part of the lower basal plate and the cell housing part of the upper basal plate in horizontal direction and in vertical direction (distance between the cell culture membrane of the upper basal plate and the cell culture membrane of the lower basal plate) can be easily and accurately performed, and the operability when culturing is enhanced.

### <Production method of the cell culture device of the first invention>

Next, the production method of the cell culture device of the first invention is explained.

The production method of the lower basal plate and the upper basal plate in the cell culture device of the first invention is not particularly limited, and they can be fabricated by various methods, while it is preferred to fabricate them by photolithography which is a semiconductor processing technique. For example, a method similar as the cell culture sheet described in Japanese Unexamined Patent Application Publication No. 2014-147342 can be used.

In the following, one example of production method of the cell culture device of the first invention of the present invention is explained.

Specifically, the case where in the cell culture device of the present invention, the lower basal plate comprises a plurality of cell culture parts arranged in a grid pattern, and the upper basal plate comprises a plurality of cell housing parts arranged in a grid pattern, the upper part of the lower partitioning member separating the plurality of grid-patterned cell culture parts of the lower basal plate constitutes a convex part, and a concave part corresponding to the upper convex part of the lower partitioning member is formed on the lower part of the upper partitioning member, corresponding to the lower partitioning member, separating the plurality of cell housing parts of the upper basal plate is explained. Further, the case where silicon wafers with a (100) crystal orientation on the surface and a mirror surface on one side are used as the lower basal plate and the upper basal plate is explained.

### (Fabrication of the lower basal plate)

The lower basal plate fabrication process comprises a cell culture part formation process and a bottom membrane micropore formation process.

Examples of the cell culture part formation process include one that involves a step of forming a silicon nitride membrane (inorganic material membrane) as transparent inorganic material on both surfaces of a silicon wafer using chemical vapor deposition such as plasma CVD method, etc.; a step of coating photoresist (photosensitive resin) on the silicon nitride membrane on one surface (non-mirror surface) of the silicon wafer to form a resist layer, and irradiating the resist layer surface with ultraviolet light via a photomask on which a plurality of patterns for etching windows are drawn; a step of immersing the silicon wafer in a developing solution to develop the resist layer and patterning the etching windows; a step of etching the exposed part of the silicon nitride membrane using a plasmarized reactive gas with the remaining resist layer as a mask (protection membrane), and then removing the resist layer using an organic solvent; and a step of immersing the silicon wafer in an etchant and etching the silicon in a truncated quadrangular shape (inverted pyramid shape) along the crystal plane of silicon to form a plurality of cell culture parts in a grid pattern. Further, at the same time of forming the cell culture part, a grid-patterned convex part (peripheral wall of the cell culture part) is formed.

Further, examples of the bottom membrane micropore formation process include one that involves a step of forming a resist layer by coating photoresist (photosensitive resin) on the silicon nitride membrane on the other surface (mirror surface) of the silicon wafer, and irradiating it with ultraviolet light via a glass mask on which a plurality of micropores per one cell culture part are drawn; a step of immersing the silicon wafer in a developing solution to develop the resist layer, and patterning micropores; and a step of forming micropores by etching the exposed part of the silicon nitride membrane using a plasmarized reactive gas with the remaining resist layer as a mask (protection membrane), and then removing the resist layer.

Further, it is preferable to comprise a finishing step of finally immersing the silicon wafer in an etchant to etch the remaining silicon part along the crystal plane of silicon from the etching windows and the micropores.

### (Fabrication of the upper basal plate)

The upper basal plate fabrication process comprises a cell housing part formation process and a bottom micropore and concave part formation process.

The cell housing part formation process, for example, comprises a step of forming a silicon nitride membrane (inorganic material membrane) as transparent inorganic material on both surfaces of the silicon wafer using chemical vapor deposition such as plasma CVD method, etc.; a step of coating photoresist (photosensitive resin) on the silicon nitride membrane on one side (non-mirror surface) of the silicon wafer to form a resist layer, and irradiating the resist layer surface with ultraviolet light via a photomask on which a plurality of rectangle patterns for etching windows are drawn; a step of immersing the silicon wafer in a developing solution to develop the resist layer, and patterning the etching windows; a step of etching the exposed part of the silicon nitride membrane using plasmarized reactive gas with the remaining resist layer as a mask (protection membrane), and then removing the resist layer using an organic solvent; and a step of immersing the silicon wafer in an etchant and etching silicon in a truncated quadrangular shape (inverted pyramid shape) along the crystal plane of silicon from the etching windows to form a plurality of cell housing parts arranged in a grid pattern. Further, as with the lower basal plate, a grid-patterned convex part (peripheral wall of the cell housing part) is formed at the same time of forming the cell housing part.

Further, the bottom membrane micropore and concave part formation process comprises a step of coating photoresist (photosensitive resin) on the silicon nitride membrane of the other surface (mirror surface) of the silicon wafer to form a resist layer, and irradiating it with ultraviolet light via a photomask on which one micropore, per one cell housing part, and a concave part pattern are drawn; a step of immersing the silicon wafer in a developing solution to develop the resist layer to pattern the micropores and the concave part; a step of etching the exposed part of the silicon nitride membrane using plasmarized reactive gas with the remaining resist layer as a mask (protection membrane) to form the micropores and the concave part, and then removing the resist layer with an organic solvent, and a step of immersing the silicon wafer in an etchant with added surfactant to etch the silicon along the crystal plane of silicon and form the concave part.

Meanwhile, it is preferable to perform a finishing treatment of etching remaining silicon parts in the cell housing formation process at the same time as the treatment of forming the concave part.

### <Cell culture method of the first invention>

Next, the cell culture method using the cell culture device of the above first invention is explained.

The cell culture method of the first invention is a cell culture method of culturing cells using the cell culture device of the above first invention, and is characterized in that cells are seeded on the bottom membrane of the cell housing part of the upper basal plate, allowing a cell to drop onto the bottom membrane of the cell culture part of the lower basal plate through one micropore formed in the bottom membrane of the cell housing part of the upper basal plate, and culturing the cell in the cell culture part.

According to the cell culture method of the first invention, since cells are dropped and seeded onto the cell culture part of the lower basal plate through one micropore formed in the bottom membrane of the cell housing part of the upper basal plate, it is possible to introduce one cell into one well at a higher probability as compared to a conventional method of seeding directly cells to a microwell array using a micropipette. Further, adherent cells such as neurons require a large culture area, and one cell can be introduced at a high probability even to such a large cell culture part.

In the following, the embodiments of the cell culture device for one embodiment of the first invention will be specifically explained by referring to drawings, but the present invention is not limited to these embodiments.

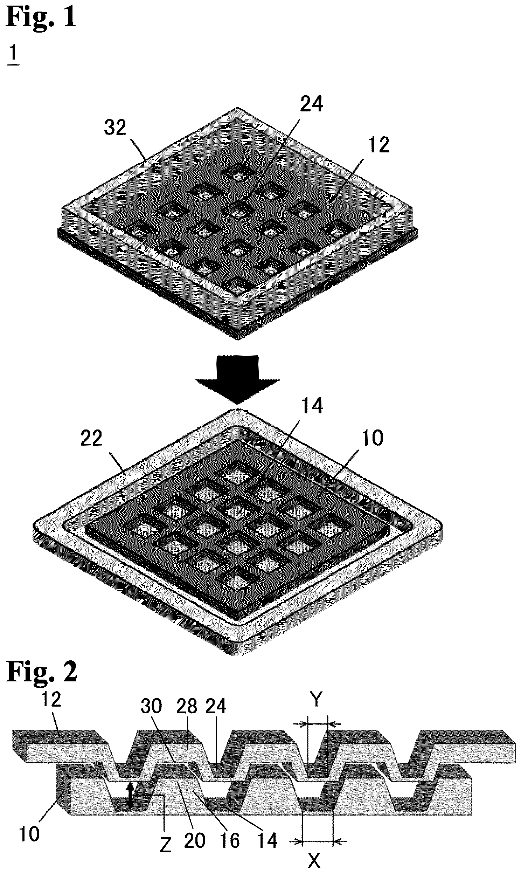

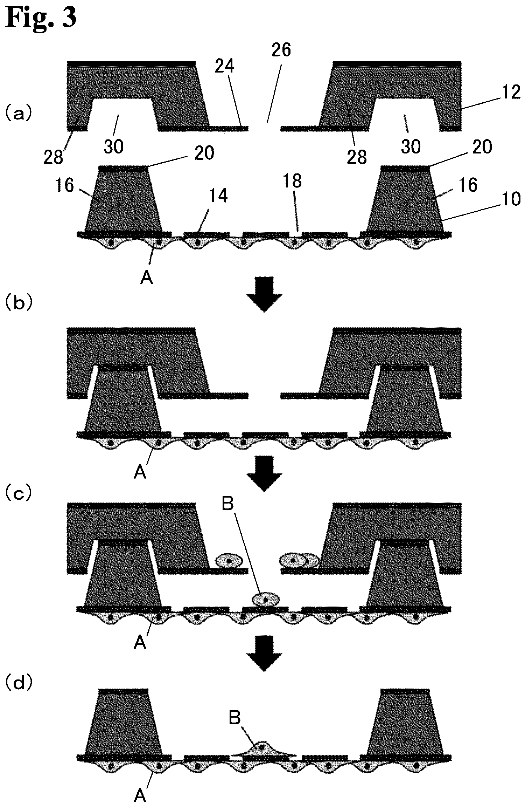

Here, Fig. 1 is a brief explanation drawing of a cell culture device for one embodiment of the first invention. Fig. 2 is a brief cross-section view of the lower basal plate and the upper basal plate of the cell culture device of Fig. 1. Fig. 3 is an explanation drawing of the cell culture method using the cell culture device of Fig. 1.

As shown in Fig. 1, the cell culture device 1 for one embodiment of the first invention comprises a square lower basal plate 10, and a square upper basal plate 12 arranged on the upper side of the lower basal plate 10.

As shown in Fig. 2 and Fig. 3, the lower basal plate 10 is arranged in a grid pattern of 4 × 4, and comprises 16 cell culture parts 14 having bottom membranes of 300 µm square (X). The bottom membrane of the cell culture part 14 is a cell culture membrane made of silicon nitride having about 200 micropores 18 with a diameter of about 3 µm. The upper part of the lower partitioning member 16 separating the grid-patterned cell culture parts 14 of the lower basal plate 10 constitutes the convex part 20. The lower basal plate 10 comprises a PC (polycarbonate) holder 22 as a support frame part supporting the lower basal plate 10.

As shown in Fig. 2 and Fig. 3, the upper basal plate 12 is arranged in a grid pattern of 4 × 4, and comprises 16 cell housing parts 24 having bottom membranes of 150 µm square (Y). The bottom membrane of the cell housing part 24 is a silicon nitride membrane in the center of which one micropore 26 with a diameter of about 12 µm is formed. On the lower part of the upper partitioning member 28 separating the cell housing parts 24 of the upper basal plate 12, the concave part 30 corresponding to the upper convex part 20 of the lower partitioning member 16 is formed. The distance (Z) between the bottom part of the cell culture part 14 of the lower basal plate 10 and the cell housing part 24 of the upper basal plate 12 is about 150 µm, and the position adjustment in horizontal direction and in vertical direction is performed by the mating of the upper convex part 20 of the lower basal plate 10 and the concave part 30 of the upper basal plate 12. The upper basal plate 12 comprises a PDMS (polydimethylsiloxane) frame 32 as a support frame part supporting the upper basal plate 12.

Next, one example of the cell culture method using the above-mentioned cell culture device 1 is explained.

As shown in Fig. 3, functional molecules are first modified on the upper surface of the cell culture part 14 of the lower basal plate 10, while functional molecules are modified and astrocytes A are seeded on the lower surface of the cell culture part 14 of the lower basal plate 10 (Fig. 3 (a)).

Then, the upper basal plate 12 is overlayed on the lower basal plate 10 (Fig. 3 (b)). At that time, the position adjustment in horizontal direction and in vertical direction is performed by mating the convex part 20 of the lower basal plate 10 and the concave part 30 of the upper basal plate 12, and contacting the top surface of the convex part 20 of the lower basal plate 10 with the bottom surface of the concave part 30 of the upper basal plate 12.

Then, neurons B are seeded by introducing a cell suspension of neurons into the cell housing part 24 of the upper basal plate 12 (Fig. 3 (c)). This allows one neuron B to be introduced into the cell culture part 14 of the lower basal plate 10 at a high probability via the micropore 26 of the bottom membrane of the cell housing part 24.

Then, the cell culture device 1 is placed in the incubator, and the neuron B which has passed through the micropore 26 of the upper basal plate 12 and reached the lower basal plate 10 is attached to the surface of the cell culture part 14 of the lower basal plate 10.

Then, the upper basal plate 12 is removed, and culturing is continued in the lower basal plate 10 (Fig. 3 (d)).

As stated above, the cell culture device of the first invention enables a single cell to be seeded in the cell culture part of the lower basal plate at a high probability by allowing a cell to drop through one micropore that the cell housing part of the upper basal plate has. Further, the single neuron B and the astrocytes A are co-cultured so as to sandwich the bottom membrane (cell culture membrane) of the cell culture part 14 of the lower basal plate 10, and the cell-to-cell communication of the both cells through the micropores 18 is realized. This allows long-term culture of a single neuron, and enables analysis and evaluation of drug efficacy at a single neuron level.

### <Cell culture device of the second invention>

Next, the cell culture device of the second invention is explained.

The cell culture device of the second invention is characterized in that it comprises a lower basal plate comprising a cell culture part having a bottom membrane (cell culture membrane) in which a plurality of micropores are formed, and an upper basal plate arranged on the upper side of the lower basal plate, and comprising a cell housing part having a bottom membrane (cell culture membrane) in which a plurality of micropores are formed, and when the upper basal plate is arranged on the lower basal plate, the bottom membrane of the lower basal plate and the bottom membrane of the upper basal plate are arranged spaced apart up and down by a predetermined distance.

In the cell culture device of the second invention, since the cell culture membrane of the lower basal plate and the cell culture membrane of the upper basal plate are arranged spaced apart up and down by a predetermined distance, a good cell-to-cell communication can be realized by culturing cells on one surface or both surfaces of each cell culture membrane of the upper basal plate and the lower basal plate in advance, and by overlaying these basal plates. Further, it is possible to easily overlay the basal plates in three or more layers for culturing cells.

The lower basal plate of the cell culture device of the second invention is similar to the lower basal plate of the cell culture device of the first invention (in the case where a plurality of micropores are formed in the bottom membrane of the cell culture part). The upper basal plate of the cell culture device of the second invention is similar to the upper basal plate of the cell culture device of the first invention, wherein the bottom membrane is made as a bottom membrane in which a plurality of micropores are formed in the lower basal plate of the cell culture device of the first invention.

Further, the production of the cell culture device of the second invention is almost similar to the production method of the cell culture device of the first invention, and the changes in the production method based on the changes of configuration of the above-mentioned device can be employed by appropriately applying the production method of the first invention.

### <Cell culture method of the second invention>

Next, the cell culture method using the cell culture device of the above second invention is explained.

The cell culture method of the second invention is a cell culture method for culturing cells using the cell culture device of the above second invention, which is characterized by culturing cells on one surface or both surfaces of the cell culture membrane of the lower basal plate, and culturing cells on one surface or both surfaces of the cell culture membrane of the upper basal plate.

According to the cell culture method of the second invention, by culturing cells on one surface or both surfaces of each cell culture membrane of the upper basal plate and the lower basal plate in advance, and by using the cell culture device wherein the upper basal plate is arranged on the lower basal plate, it is easily possible to culture three layers or four layers of cells.

In the following, the embodiments of the cell culture device for one embodiment of the second invention will be specifically explained by referring to drawings, while the present invention is not limited to these embodiments.

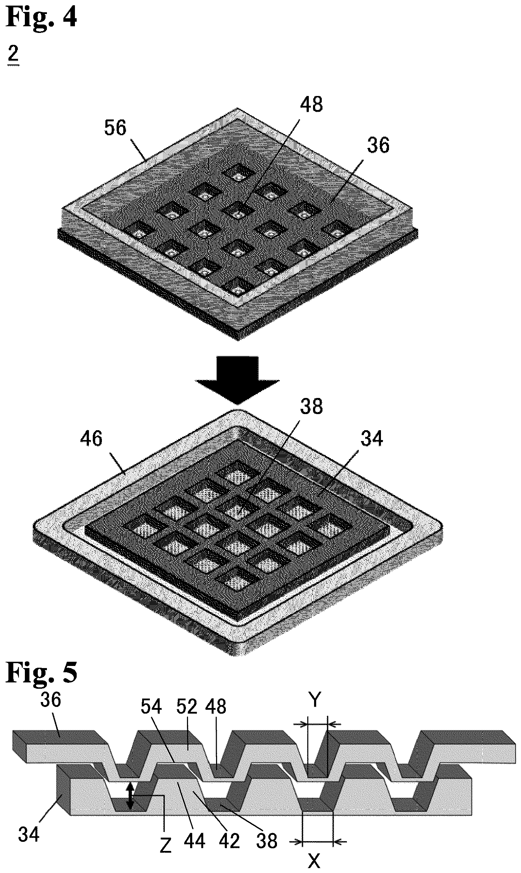

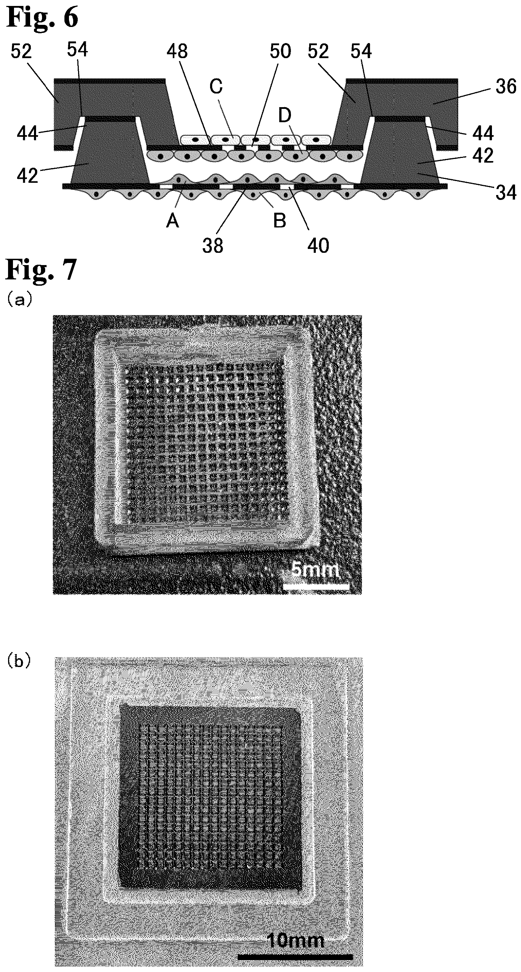

Here, Fig. 4 is a brief explanation drawing of a cell culture device for one embodiment of the second invention. Fig. 5 is a brief cross-section view of the lower basal plate and the upper basal plate of the cell culture device in Fig. 4. Fig. 6 is an explanation drawing of the cell culture method using the cell culture device in Fig. 4.

As shown in Fig. 4, the cell culture device 2 for one embodiment of the second invention comprises a square lower basal plate 34 and a square upper basal plate 36 arranged on the upper side of the lower basal plate 34.

As shown in Fig. 5 and Fig. 6, the lower basal plate 34 is arranged in a grid pattern of 4 × 4, and comprises 16 cell culture parts 38 having bottom membranes of 300 µm square (X). The bottom membrane of the cell culture part 38 is a cell culture membrane made of silicon nitride having about 200 micropores 40 with a diameter of about 3 µm. The upper part of the lower partitioning member 42 separating the grid patterned cell culture parts 38 of the lower basal plate 34 constitutes the convex part 44. The lower basal plate 34 comprises a PC (polycarbonate) holder 46 as a support frame part supporting the lower basal plate 34.

As shown in Fig. 5 and Fig. 6, the upper basal plate 36 is arranged in a grid pattern of 4 x 4, and comprises 16 cell culture parts (cell housing parts) 48 having bottom membranes of 150 µm square (Y). The cell culture part 48 is constituted of a cell culture membrane made of silicon nitride having about 200 micropores 50 with a diameter of about 3 µm. On the lower part of the upper partitioning member 52 separating the 16 cell culture parts 48 of the upper basal plate 36, the concave part 54 corresponding to the upper convex part 44 of the lower partitioning member 42 is formed. The distance (Z) between the bottom membrane of the cell culture part 38 of the lower basal plate 34 and the bottom membrane of the cell culture part 48 of the upper basal plate 36 is about 150 µm, and the position adjustment in horizontal direction and in vertical direction is performed by mating the upper part convex part 44 of the lower basal plate 34 with the concave part 54 of the upper basal plate 36. The upper basal plate 36 comprises a PDMS (polydimethylsiloxane) frame 56 as a support frame part supporting the upper basal plate 36.

Next, one example of the cell culture method using the above-mentioned cell culture device 2 is explained.

First, functional molecules are modified on the upper surface of the bottom membrane of the cell culture part 38 of the lower basal plate 34, and astrocytes A are seeded and attached to it. Further, functional molecules are modified on the lower surface of the bottom membrane of the cell culture part 38 of the lower basal plate 34, and neurons B are seeded and attached.

Similarly, pericytes D are attached to the lower surface of the bottom membrane of the cell culture part 38 of the upper basal plate 36, and vascular endothelial cells C are attached to the upper surface.

Next, as shown in Fig. 6, the upper basal plate 36 is overlayed on the lower basal plate 34. At that time, the position adjustment in horizontal direction and in vertical direction is performed by mating the convex part 44 of the lower basal plate 34 with the concave part 54 of the upper basal plate 36, and contacting the top surface of the convex part 44 of the lower basal plate 34 with the bottom surface of the concave part 54 of the upper basal plate 36.

As stated above, by co-culturing neurons B and astrocytes A on both surfaces of the bottom membrane of the cell culture part 38 of the lower basal plate 34, it is possible to reconstruct the double layer structure of the nerve tissue in the brain. Further, by co-culturing vascular endothelial cells C and pericytes D on both surfaces of the bottom membrane of the cell culture part 48 of the upper basal plate 36, it is possible to reconstruct the blood vessel system in the brain. Further, by arranging the cell culture membranes of the lower basal plate 34 and the upper basal plate 36 in extremely close proximity, communication between these cells can be promoted. The construction of such a blood-brain barrier model makes it possible to analyze drug transfer from blood to brain tissue, etc.

As such, by using the cell culture device of the second invention, it is easily possible to culture cells in multilayers by culturing cells on both surfaces of each cell culture membrane of the upper basal plate and the lower basal plate in advance, and arranging the upper basal plate on the lower basal plate.

### Examples

In the following, the present invention will be explained in further details based on Examples, while the present invention is not limited to these Examples.

### [Fabrication of the lower basal plate]

By using a silicon wafer (about 50 mm diameter, about 300 µm thickness) with a mirror surface on one side and a crystal orientation (100) on the surface, the lower basal plate was fabricated according to the following procedure.

(1) A silicon nitride membrane (SiN membrane) with a thickness of 1.2 µm was formed on both surfaces of the silicon wafer using plasma CVD.
(2) Positive resist OFPR-800LB 23 cp (manufactured by TOKYO OHKAKOGYO CO., LTD.) was coated on the non-mirror surface of the silicon wafer, and ultraviolet light was irradiated onto the resist surface via a glass mask on which 16 × 16 rectangular patterns (690 µm on a side) for etching windows are drawn.
(3) The silicon wafer was immersed in a developing solution NMD-3 (2.38% tetramethylammonium hydroxide solution; manufactured by TOKYO OHKA KOGYO CO., LTD.) to develop the resist and pattern the etching windows.

(4) After the exposed part of the SiN membrane was etched with CF₄ gas plasma using the remaining resist layer as a mask, the resist was removed using acetone.
(5) The silicon wafer was immersed in a 25% tetramethylammonium hydroxide solution heated to about 80°C, and the silicon was etched to a depth of about 200 µm in an inverted truncated quadrangular shape (inverted pyramid shape) along the crystal plane of silicon.
(6) Positive resist OFPR-800LB 23 cp was coated on the mirror surface side of the silicon wafer, and ultraviolet light was irradiated onto the resist surface via a glass mask on which 208 micropores (2 µm diameter) per one cell culturing part are drawn.
(7) The silicon wafer was immersed in a developing solution NMD-3 to develop the resist and pattern the micropores.

(8) After the exposed part of the SiN membrane was etched with CF₄ gas plasma using the remaining resist layer as a mask, the resist was removed using acetone.
(9) The silicon wafer was immersed in a 25% tetramethylammonium hydroxide solution heated to about 80°C, and the silicon was etched along the crystal plane of silicon from the etching windows and the micropores, to form self-supported membranes (bottom membranes) made of SiN and form cell culture parts comprising peripheral walls (convex part). The final thickness of the bottom membrane was about 1 µm, the final diameter of the micropores was about 3.5 µm, and the size of the bottom membrane per one cell culturing part was 300 µm square.
(10) A separately fabricated rectangular holder made of polycarbonate was bonded with PDMS to the outer periphery of the basal plate that was cut from the silicon wafer.

### [Fabrication of the upper basal plate]

Next, by using a silicon wafer (about 50 mm diameter, about 300 µm thickness) with a mirror surface on one side and a crystal orientation (100) on the surface, the upper basal plate was fabricated according to the following procedure.

(1) A SiN membrane with a thickness of 1.2 µm was formed on both surfaces of the silicon wafer using plasma CVD.
(2) Positive resist OFPR-800LB 23 cp was coated on the non-mirror surface of the silicon wafer, and ultraviolet light was irradiated onto the resist surface via a glass mask on which 16 x 16 rectangular patterns (570 µm on a side) for etching windows are drawn.
(3) The silicon wafer was immersed in a developing solution NMD-3 to develop the resist and pattern the etching windows.

(4) After the exposed part of the SiN membrane was etched with CF₄ gas plasma using the remaining resist layer as a mask, and the resist was removed using acetone.
(5) The silicon wafer was immersed in a 25% tetramethylammonium hydroxide solution heated to about 80°C, and the silicon was etched to a depth of about 180 µm in an inverted truncated quadrangular shape (inverted pyramid shape) along the crystal plane of silicon.
(6) Positive resist OFPR-800LB 23 cp was coated on the mirror surface of the silicon wafer, and ultraviolet light was irradiated onto the resist surface via a glass mask on which one micropore (11 µm diameter) and a pattern of concave part are drawn.
(7) The silicon wafer was immersed in a developing solution NMD-3 to develop the resist and pattern the micropore and the concave part.

(8) After the exposed part of the SiN membrane was etched with CF₄ gas plasma using the remained resist layer as a mask, the resist was removed using acetone.
(9) The silicon wafer was immersed in a 25% tetramethylammonium hydroxide solution heated to about 80°C, and etched along the crystal plane of silicon, until the depth of the concave part became about 150 µm. At that time, the silicon etching also proceeded from the etching windows and micropores, forming a self-supported membrane made of SiN. Further, the surfactant Triton X-100 at a concentration of 0.01% was added to the etching solution in advance to suppress overetching of the corners constituting the concave part. The final thickness of the bottom membrane was about 1 µm, the final diameter of the micropore was about 13 µm, and the size of the bottom membrane per one cell housing part was 150 µm square.
(10) A rectangular frame made of PDMS (polydimethylsiloxane) was bonded to the outer periphery of the upper surface that was cut from the silicon wafer. The bonding was performed by irradiating the SiN membrane surface and the PDMS surface of the upper basal plate surface with vacuum ultraviolet light to form OH groups on both surfaces, and then by dehydration condensation reaction when both surfaces were contacted.

The actual photograph of the fabricated cell culture device is shown in Fig. 7. In the fabricated cell culture device, it was confirmed that the position adjustment of the cell culture parts of the lower basal plate and the cell housing parts of the upper basal plate can be easily performed by mating the grid-patterned convex part of the lower basal plate (Fig. 7 (b)) and the grid-patterned concave part of the upper basal plate (Fig. 7 (a)). The distance between the bottom membrane of the cell culture part of the lower basal plate and the bottom membrane of the cell housing part of the upper basal plate was about 150 µm.

### [Cell seeding test]

The number of cells seeded into the cell culture parts of the lower basal plate through the micropores formed in the SiN membranes of the upper basal plate was confirmed using the cell culture device fabricated in the above Example.

### (Preparation of the cell culture device and a cell suspension)

By immersing the lower basal plate overnight at room temperature in a PDL (Poly-D-lysine) aqueous solution prepared to a concentration of 0.1 mg/ml with sterile water, PDL was modified on the SiN membrane surface. By storing iMatrix-511 (manufactured by Matrixome Inc.) prepared in PBS (phosphate buffer saline) to the surface density of 0.5 µm/cm² on the surface of the lower basal plate, and placing it in an incubator of 37°C and 5% CO₂ concentration, Laminin-511 was modified on PDL of the SiN membrane surface. Strip-shaped spacers (about 1 mm thickness) made of PDMS were placed on the bottom surface of a 35 mm dish, the lower basal plate was placed on them, and a medium of Neurobasal Plus Medium (manufactured by Life Technologies Corporation) containing B-27 Plus Supplement (manufactured by Life Technologies Corporation) at a concentration of 2% was introduced until the lower basal plate was completely immersed. The upper basal plate was overlayed on the lower basal plate. At that time, position adjustment was performed by mating the grid-patterned convex part of the lower basal plate and the grid-patterned concave part of the upper basal plate, and contacting the top surface of the convex part of the lower basal plate with the bottom surface of the concave part of the upper basal plate. The above procedure was repeated, and 3 similar cell culture devices were prepared.

Hippocampal neurons collected from a mouse were suspended in a medium, and 3 different concentrations of cell suspensions were prepared so that seeding densities were 1800 cells/cm², 9000 cells/cm², and 18000 cells/cm².

### (Cell seeding test)

(1) Neurons were seeded by introducing 3 different cell suspensions into the PDMS frame of each upper basal plate of the 3 devices.
(2) The devices were placed in an incubator at 37°C and a CO₂ concentration of 5% for 2 hours to wait for the neurons that had passed through the micropores of the upper basal plate and reached the lower basal plate to adhere to the SiN membrane surface of the lower basal plate.
(3) The upper basal plate was removed, and the lower basal plate was washed with PBS.
(4) The lower basal plate was immersed in a 4% paraformaldehyde-phosphate buffer solution for 15 min for cell fixation treatment, and then washed with PBS.
(5) The lower basal plate was immersed in a 0.1% Triton X-100/PBS solution for 15 min for permeabilization treatment, and then washed with PBS.
(6) The lower basal plate was immersed for 30 min at room temperature in a DAPI solution diluted 500-fold with PBS to fluorescently stain the cell nuclei of neurons attached to the SiN membrane surface of the lower basal plate, and then washed with PBS.
(7) The cell nuclei stained with DAPI were counted by observation using a fluorescence inverted microscope to obtain the number of neurons in each well (in the cell culture part).

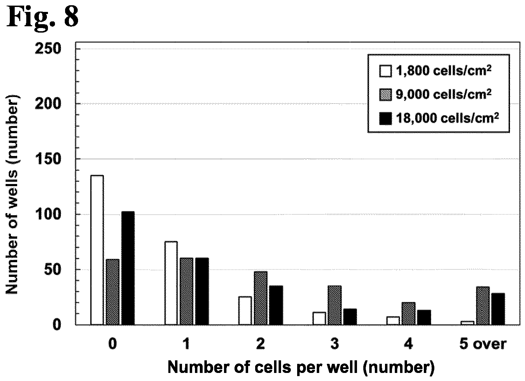

The results of cell seeding test are shown in Fig. 8.

As shown in Fig. 8, the number of wells (number of cell culture parts) in which a single neuron adhered was about 20% of the total. Since it was a few % in the case of a conventional method, it can be seen that the effect of using the cell culture device of the present invention is significant. Further, it was found that the number of wells in which a single neuron was obtained does not depend on the seeding density of neurons. Generally, when seeding cells in a culture vessel, the density of cells differs between the center part and the periphery part of the vessel due to the significant influence of the meniscus of solution at the outer periphery of the vessel. Particularly, when a culture vessel is smaller, the influence is larger. However, it was found that the cell culture method using the cell culture device of the present invention enables cell seeding without being affected by the meniscus of solution.

### Industrial Applicability

The cell culture device of the present invention is industrially useful because it can be used for cell culture.

### Reference Signs List

- 1: cell culture device (first invention)
- 2: cell culture device (second invention)
- 10: lower basal plate
- 12: upper basal plate
- 14: cell culture part
- 16: lower partitioning member
- 18: micropore
- 20: convex part
- 22: PC holder
- 24: cell housing part
- 26: micropore
- 28: upper partitioning member
- 30: concave part
- 32: PDMS frame
- 34: lower basal plate
- 36: upper basal plate
- 38: cell culture part
- 40: micropore
- 42: lower partitioning member
- 44: convex part
- 46: PC holder
- 48: cell culture part
- 50: micropore
- 52: upper partitioning member
- 54: concave part
- 56: PDMS frame
- A: astrocyte
- B: neuron
- C: vascular endothelial cell
- D: pericyte

## Claims

1. A cell culture device comprising:
a lower basal plate comprising a cell culture part having a bottom membrane, and
an upper basal plate arranged on an upper side of the lower basal plate, and comprising a cell housing part having a bottom membrane in which a micropore is formed,
wherein when arranging the upper basal plate on the lower basal plate, the bottom membrane of the lower basal plate and the bottom membrane of the upper basal plate are arranged spaced apart up and down by a predetermined distance.

2. The cell culture device according to claim 1, wherein one micropore is formed in the bottom membrane of the upper basal plate.

3. The cell culture device according to claim 2, wherein a plurality of micropores are formed in the bottom membrane of the lower basal plate.

4. The cell culture device according to claim 2, wherein the bottom membrane of the upper basal plate and/or the lower basal plate is constituted of a transparent material.

5. The cell culture device according to claim 4, wherein the bottom membrane of the upper basal plate and/or of the lower basal plate comprises at least one inorganic material selected from silicon nitride (SiN), silicon oxide (SiO₂), and silicon oxynitride (SiON).

6. The cell culture device according to claim 2, wherein the lower basal plate comprises a convex part or a concave part, and the upper basal plate comprises a concave part or a convex part, wherein the convex part or the concave part of the lower basal plate and the concave part or the convex part of the upper basal plate are mated to perform position adjustment of the bottom membrane of the lower basal plate and the bottom membrane of the upper basal plate.

7. The cell culture device according to claim 2, wherein the lower basal plate comprises a plurality of cell culture parts arranged in a grid pattern, and the upper basal plate comprises a plurality of cell housing parts arranged in a grid pattern.

8. The cell culture device according to claim 7, wherein the upper part of a lower partitioning member that divides the plurality of grid-patterned cell culture parts of the lower basal plate constitutes a convex part, and a concave part corresponding to the upper convex part of the lower partitioning member is formed on the lower part of an upper partitioning member, corresponding to the lower partitioning member, that divides the plurality of cell housing parts of the upper basal plate.

9. The cell culture device according to claim 1, wherein a plurality of micropores are formed in the bottom membrane of the cell culture part of the lower basal plate, and a plurality of micropores are formed in the bottom membrane of the cell housing part of the upper basal plate.

10. The cell culture device according to claim 9, wherein the bottom membrane of the upper basal plate and/or the lower basal plate is constituted of a transparent material.

11. The cell culture device according to claim 10, wherein the bottom membrane of the upper basal plate and/or the lower basal plate comprises at least one inorganic material selected from silicon nitride (SiN), silicon oxide (SiO₂), and silicon oxynitride (SiON).

12. The cell culture device according to claim 9, wherein the bottom membrane of the cell culture part of the lower basal plate and the bottom membrane of the cell housing part of the upper basal plate are arranged spaced apart up and down by a distance of 5 to 500 µm.

13. The cell culture device according to claim 9, wherein the lower basal plate comprises a convex part or a concave part, and the upper basal plate comprises a concave part or a convex part, wherein the convex part or the concave part of the lower basal plate and the concave part or the convex part of the upper basal plate are mated to perform position adjustment of the bottom membrane of the lower basal plate and the bottom membrane of the upper basal plate.

14. The cell culture device according to claim 9, wherein the lower basal plate comprises a plurality of cell culture parts arranged in a grid pattern, and the upper basal plate comprises a plurality of cell housing parts arranged in a grid pattern.

15. The cell culture device according to claim 14, wherein the upper part of a lower partitioning member that divides the plurality of grid-patterned cell culture parts of the lower basal plate constitutes a convex part, and a concave part corresponding to the upper convex part of the lower partitioning member is formed on the lower part of an upper partitioning member, corresponding to the lower partitioning member, that divides the plurality of cell housing parts of the upper basal plate.

16. The cell culture device according to claim 1, 2 or 9, wherein the lower basal plate and the upper basal plate are fabricated by using photolithography which is a semiconductor processing technique.

17. A cell culture method comprising culturing cells by using the cell culture device according to claim 2, comprising seeding cells on the bottom membrane of the cell housing part of the upper basal plate, allowing a cell to drop on the bottom membrane of the cell culture part of the lower basal plate, through one micropore formed in the bottom membrane of the cell housing part of the upper basal plate, to culture the cell at the cell culture part.

18. A cell culture method comprising culturing cells by using the cell culture device according to claim 9, comprising culturing cells on one surface or both surfaces of the cell culture membrane of the lower basal plate, and culturing cells on one surface or both surfaces of the cell culture membrane of the upper basal plate.
